# EUROPEAN PATENT SPECIFICATION

(11) **EP 1 652 834 B1**
(45) Date of publication and mention of the grant of the patent: **01.10.2008**
(21) Application number: 05023274.3
(22) Date of filing: 25.10.2005
(51) Int. Cl.: C07C 67/60, C07C 69/88

(54) **Process for producing salicylic esters**
Verfahren zur Herstellung von Salicylestern
Procedé de production d'esters salicyliques

(30) Priority: 29.10.2004 JP 2004315570
(43) Date of publication of application: 03.05.2006
(73) Proprietor: KAO CORPORATION, Chuo-ku Tokyo (JP)
(72) Inventor: Ohno, Satoshi, Wakayama-shi Wakayama (JP); Uehara, Takefumi, Wakayama-shi Wakayama (JP); Kotachi, Shinji, Wakayama-shi Wakayama (JP); Tanaka, Shigeyoshi, Wakayama-shi Wakayama (JP)
(74) Representative: HOFFMANN EITLE

(56) References cited:
- FR-A- 2 733 981
- US-A- 2 787 620
- US-A- 4 594 444

## Description

### FIELD OF THE INVENTION

The present invention relates to a process for producing salicylic esters by transesterification reaction in an industrially useful manner.

### BACKGROUND OF THE INVENTION

Salicylic esters have been used in various applications such as, for example, perfumes, ultraviolet absorbers and developers for thermosensible papers. Examples of the salicylic esters which have been practically used as perfumes include methyl salicylate, ethyl salicylate, butyl salicylate, isobutyl salicylate, pentyl salicylate, isopentyl salicylate, hexyl salicylate, cis-3-hexenyl salicylate, cyclohexyl salicylate and phenylethyl salicylate. Also, examples of the salicylic esters used as ultraviolet absorbers include phenyl salicylate, p-tert-butylphenyl salicylate and p-octylphenyl salicylate.

As known in the art, the salicylic esters have been conventionally produced by the direct esterification method in which salicylic acid is directly reacted with alcohols or phenols, or the transesterification method in which a salicylic lower alkyl ester such as methyl salicylate is transesterified with alcohols or phenols.

In the transesterification reactions for producing the salicylic esters, there have been generally used transesterification catalysts. For example, there are disclosed the methods using sodium methoxide as the transesterification catalyst (e.g., refer to JP 60-160040A), and the methods using potassium carbonate as the transesterification catalyst (e.g., refer to BR-A-8905636). However, in these conventional methods using the basic catalysts, it is inevitably required that the resultant reaction solution is subjected to water-washing treatment in a post treatment process thereof in order to recover the aimed salicylic esters therefrom at a high efficiency while suppressing occurrence of side reactions. On the other hand, as the processes for production of the salicylic esters which require no water-washing treatment, there are known transesterification reaction methods using, for example, a titanium catalyst (e.g., refer to FR-A-2733981). However, in the transesterification reactions using such a titanium catalyst, a small amount of salicylic acid is by-produced during the reaction or distillation process. The salicylic acid having a melting point as high as about 160°C tends to be precipitated in apparatuses used, for example, subsequent to the distillation process, thereby causing undesirable problems such as deteriorated cooling efficiency of a condenser and clogging in conduits as well as a large load for cleaning facilities after completion of the production owing to these problems.

### SUMMARY OF THE INVENTION

The present invention provides a process for producing a salicylic ester which comprises the step of subjecting a transesterification reaction solution obtained by transesterifying a salicylic lower alkyl ester with alcohols or phenols in the presence of a transesterification reaction catalyst as defined below to distillation treatment in the presence of or an epoxy compound having a boiling point higher than that of the salicylic ester as produced.

### DETAILED DESCRIPTION OF THE INVENTION

The present invention is directed to an industrially useful process for producing salicylic esters by transesterification reaction in which precipitation of salicylic acid in apparatuses is effectively suppressed in processes subsequent to distillation process thereof, so that problems such as deteriorated cooling efficiency of a condenser and clogging in conduits due to the precipitation of salicylic acid as well as a large load for cleaning facilities after completion of the production owing to these problems can be prevented.

In the process for producing the salicylic ester according to the present invention, there is used the method in which a salicylic lower alkyl ester is transesterified with alcohols or phenols in the presence of a transesterification reaction catalyst.

The salicylic lower alkyl ester usable in the present invention is a compound represented by the general formula (6): 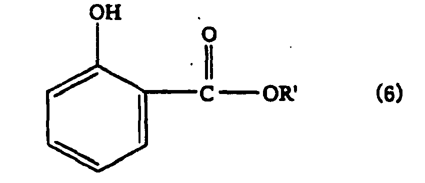 wherein R' is a linear or branched alkyl group having 1 to 4 carbon atoms.

Specific examples of the linear or branched alkyl group having 1 to 4 carbon atoms which is represented by R' in the above general formula (6), include methyl, ethyl, propyl, isopropyl, butyl, isobutyl, sec-butyl and tert-butyl.

As the salicylic C₁₋₄ alkyl ester, there may be generally used those esters in which the carbon number of the alkyl group in the ester moiety thereof is smaller than that of the alcohols or phenols used in the transesterification reaction. In view of the transesterification reactivity, among these esters, preferred are methyl salicylate and ethyl salicylate, and more preferred is methyl salicylate.

As the alcohols or phenols to be transesterified with the salicylic lower alkyl ester, any alcohols or phenols capable of producing the salicylic esters by transesterification reaction with the salicylic lower alkyl ester may be used without particular limitations. Examples of the alcohols or phenols usable in the present invention include compounds represented by the general formula (7):

R-OH (7)

wherein R is a saturated or unsaturated, chain-like aliphatic or cyclic aliphatic group having 3 to 20 carbon atoms, a heterocyclic group having 3 to 20 carbon atoms, an aryl group having 6 to 20 carbon atoms, or an arylalkyl group having 7 to 20 carbon atoms.

Specific examples of the saturated chain-like aliphatic alcohol represented by the above general formula (7) in which R is a saturated chain-like aliphatic group having 3 to 20 carbon atoms include propanol, butanol, isobutanol, 2-isopropoxy ethanol, pentanol, isopentanol, hexanol, 3-methyl-1-pentanol, heptanol, 2- or 3-heptanol, octanol, 2- or 3-octanol, 2-ethyl hexanol, nonanol, 2-nonanol, 3,5,5-trimethyl-1-hexanol, 2,6-dimethyl heptanol, 3,7-dimethyl-1-octanol, decanol, undecanol, 2-undecanol, dodecanol and 3,4,5,6,6-pentamethyl-2-heptanol (Kohinool: available from International Flavors & Fragrances Inc. (IFF)).

Specific examples of the unsaturated chain-like aliphatic alcohol represented by the above general formula (7) in which R is an unsaturated chain-like aliphatic group having 3 to 20 carbon atoms include prenol (3-methyl-2-buten-1-ol), 1-penten-3-ol, cis-3-hexenol (Leaf alcohol), trans-2-hexenol, trans-3-hexenol, cis-4-hexenol, 2,4-hexadien-1-ol, 1-octen-3-ol (Matsutakeol), cis-6-nonenol, 2,6-nonadienol, 1-nonen-3-ol, 9-decenol, 1-undecenol, 4-methyl-3-decen-5-ol, 3,7-dimethoxy-7-methoxy-2-octanol, citronellol (3,7-dimethyl-6-octen-1-ol), geraniol (2-trans-3,7-dimethyl-2,6-octadien-1-ol), nerol (cis-3,7-dimethyl-2,6-octadien-1-ol), lavandulol (2-isopropenyl-5-methyl-4-hexen-1-ol), isodihydrolavandulol (2-isopropyl-5-methyl-2-hexen-1-ol), nonadyl (6,8-dimethyl-2-nonanol) and farnesol (3,7,11-trimethyl-2,6,10-dodecatrien-1-ol).

Specific examples of the saturated cyclic aliphatic alcohol represented by the above general formula (7) in which R is a saturated cyclic aliphatic group having 3 to 20 carbon atoms include cyclopentanol, cyclohexanol, cyclohexyl methanol, 2-cyclohexyl ethanol, 4-isopropyl cyclohexanol, 4-tert-butyl cyclohexanol, 2-tert-butyl cyclohexanol, 4-(1-methylethyl)cyclohexanemethanol (Mayol: available from IFF), 5-methyl-2-isopropyl cyclohexanol (menthol), 3-thujanol (4-methyl-1-isopropylbicyclo[3.1.0]hexan-3-ol), α-fenchyl alcohol (fenchol: 1,3,3-trimethylbicyclo[2.2.1]heptan-2-ol), borneol . (endo-1,7,7-trimethylbicyclo[2.2.1]heptan-2-ol: 2-camphanol), isoborneol (exo-2-camphanol), 2,2,4- and/or 2,4,4-trimethyl cyclopentanol, cycloheptanol, cyclooctanol, cyclodecanol, decahydro-β-naphthol, 2,2,6-trimethylcyclohexyl-3-hexanol, trimethyl norbornane methanol (Camekol: available from IFF), α,3,3-trimethylbicyclo[2.2.1]heptane-2-methanol and isocamphyl cyclohexanol [3-(5,5,6-trimethylbicyclo[2.2.1]hept-2-yl)cyclohexanol].

Specific examples of the unsaturated cyclic aliphatic alcohol represented by the above general formula (7) in which R is an unsaturated cyclic aliphatic group having 3 to 20 carbon atoms include 2,4-dimethyl-3-cyclohexene-1-methanol (Floralol: available from IFF), carveol (1-methyl-4-isopropenyl-6-cyclohexen-2-ol), dihydrocarveol (6-methyl-3-isopropenyl cyclohexanol), perillalcohol (dihydrocuminyl alcohol), isocyclogeraniol (2,4,6-trimethylcyclohex-3-ene-1-methanol), nopol(6,6-dimethylbicyclo[3.1.1.]hept-2-ene-2-ethanol), 3,3-dimethyl-Δ²,β-norbornane-2-ethanol (Patchomint: available from IFF), cyclomethylene citronellol [3-(4-methyl-3-cyclohexenyl)butanol], ambrinol (2,5,5-trimethyl-octahydro-2-naphthol), cyclooct-4-en-ol, 2-methyl-4-(2,2,3-trimethyl-3-cyclopenten-1-yl)-2-buten-1-ol, 2-methyl-4-(2,2,3-trimethyl-3-cyclopentenyl)butanol, bacdanol (2-ethyl-4-(2,2,3-trimethyl-3-cyclopenten-1-yl)-2-buten-1-ol), sandalore [3-methyl-5-(2,2,3-trimethylcyclopent-3-en-1-yl)pentan-2-ol], methyl sandeflor [2-methyl-1-(methylbicyclo{2,2,1}hept-5-en-2-yl)penten-3-ol (Mixt.)], myrthenol (6,6-dimethylbicyclo[3.1.1]hept-2-ene-2-methanol) and pinocarveol (6,6-dimethyl-2-methylenebicyclo[3.1.1]heptan-3-ol).

Specific examples of the heterocyclic alcohol represented by the above general formula (7) in which R is a heterocyclic group having 3 to 20 carbon atoms include furfuryl alcohol, tetrahydrofuran-2-methanol, 2-thienyl carbinol and tetrahydrothiophene-2-methanol.

Specific examples of the phenols represented by the above general formula (7) in which R is an aryl group having 6 to 20 carbon atoms include phenol, cresol, p-ethyl phenol, p-tert-butyl phenol, p-octyl phenol, chavicol (p-allyl phenol), thymol (2-isopropyl-5-methyl phenol), carvacrol (5-isopropyl-2-methyl phenol), gusiacol (2-methoxy phenol), creosol (2-methoxy-4-methyl phenol), 2,6-climethoxy phenol, 4-ethyl guaiacol (2-methoxy-4-ethyl phenol), eugenol (2-methoxy-4-allyl phenol), isoeugenol (2-methoxy-4-propenyl phenol) and dihydro eugenol (2-methoxy-4-propyl phenol).

Specific examples of the arylalkyl alcohol represented by the above general formula (7) in which R is an arylalkyl group having 7 to 20 carbon atoms include benzyl alcohol, 2-phenylethyl alcohol, hydratropalcohol (2-phenylpropyl alcohol), 2-tolylethyl alcohol (Hawthanol: available from IFF), 2-phenoxy ethanol, 2-methoxy-2-phenylethyl alcohol, 1-phenyl-2-pentanol, 4-methyl-1-phenyl-2-pentanol, 3-phenylpropyl alcohol, cinnamic alcohol, 3-methyl-5-phenyl pentanol-1, anise alcohol (p-methoxybenzyl alcohol), cuminic alcohol (4-isopropenylbenzyl alcohol), p,α-dimethylbenzyl alcohol, α-phenylethyl alcohol, 2,2-dimethyl-3-phenyl propanol (Muguet alcohol) and α-amylcinnamyl alcohol.

Among the above alcohols, in the present invention, there may be suitably used those alcohols having 3 to 10 carbon atoms. Examples of the preferred alcohols having 3 to 10 carbon atoms include propanol, butanol, isobutanol, pentanol, isopentanol, hexanol, 2-ethyl hexanol, octanol, 2-isopropoxy ethanol, prenol (3-methyl-2-buten-1-ol), trans-2-hexenol, cis-3-hexenol, cyclopentanol, cyclohexanol, 5-meihyl-2-isopropyl cyclohexanol (menthol), borneol (2-camphanol), isoborneol (exo-2-camphanol), 2,2,4- and/or 2,4,4-trimethyl cyclopentanol, 4-isopropyl cyclohexanol, cyclohexyl methanol, cyclooctanol, cyclooct-4-en-ol, benzyl alcohol, 2-phenylethyl alcohol and cresol. Of these alcohols, more preferred are pentanol, isopentanol, hexanol, cis-3-hexenol and cyclohexanol.

In addition, among these alcohols, preferred are secondary alcohols, and more preferred is cyclohexanol in view of the transesterification reactivity.

The salicylic esters produced by the transesterification reaction according to the present invention are preferably those salicylic esters represented by the general formula (1): 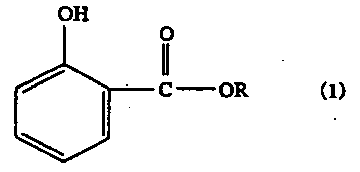 wherein R has the same meaning as defined in the above general formula (7).

Examples of the salicylic esters represented by the above general formula (1) include propyl salicylate, butyl salicylate, isobutyl salicylate, pentyl salicylate, isopentyl salicylate, hexyl salicylate, 2-ethylhexyl salicylate, octyl salicylate, 2-isopropoxyethyl salicylate, prenyl salicylate, trans-2-hexenyl salicylate, cis-3-hexenyl salicylate, cyclopentyl salicylate, cyclohexyl salicylate, esters of salicylic acid and menthol, esters of salicylic acid and borneol, esters of salicylic acid and isoborneol, 2,2,4- and/or 2,4,4-trimethylcyclopentyl salicylate, 4-isopropylcyclohexyl salicylate, cyclohexylmethyl salicylate, cyclooctyl salicylate, cyclooct-4-enyl salicylate, benzyl salicylate, 2-phenylethyl salicylate and cresyl salicylate. Among these salicylic esters, in the present invention, preferred are those salicylic esters in which R in the general formula (1) has 3 to 10 carbon atoms. In particular, more preferred are pentyl salicylate, isopentyl salicylate, hexyl salicylate, cis-3-hexenyl salicylate and cyclohexyl salicylate, and still more preferred is cyclohexyl salicylate.

In the process for production of the salicylic esters according to the present invention, upon subjecting the salicylic lower alkyl ester and the alcohols or phenols to transesterification reaction, there is used the transesterification reaction catalyst. The transesterification reaction catalyst is selected from titanium-based catalysts, tin-based catalysts and aluminum-based catalysts. Among these catalysts, preferred are titanium-based catalysts and tin-based catalysts in view of no need of water-washing treatment after completion of the reaction as well as high reactivity.

The titanium-based catalysts usable in the present invention include, for example, titanium compounds represented by the following general formula (2):

Ti(OR¹)₄ (2)

wherein R¹ is an alkyl group, a cycloalkyl group, an aryl group or an arylalkyl group,
or condensed products thereof.

Examples of the alkyl group as R¹ in the general formula (2) include linear or branched alkyl groups having 1 to 20 carbon atoms. Specific examples of the alkyl groups include methyl, ethyl, propyl, isopropyl, various butyl groups, various pentyl groups, various hexyl groups, various octyl groups, various decyl groups, various dodecyl groups, various tetradecyl groups, various hexadecyl groups and various octadecyl groups. Examples of the cycloalkyl group as R¹ in the general formula (2) include cycloalkyl groups having 5 to 20 carbon atoms which may have any substituent groups bonded to a ring thereof. Specific examples of the cycloalkyl group include cyclopentyl, cyclohexyl, methyl cyclohexyl and cyclooctyl. Examples of the aryl group as R¹ in the general formula (2) include aryl groups having 6 to 20 carbon atoms which may have any substituent groups bonded to an aromatic ring thereof. Specific examples of the aryl group include phenyl, tolyl, xylyl and naphthyl. Examples of the arylalkyl group as R¹ in the general formula (2) include arylalkyl groups having 7 to 20 carbon atoms which may have any substituent groups bonded to an aromatic ring thereof. Specific examples of the arylalkyl group include benzyl, phenethyl, methylbenzyl and naphthylmethyl. Meanwhile, the four OR¹ groups may be the same or different from each other.

Specific examples of the titanium-based compounds represented by the above general formula (2) include tetramethoxy titanium, tetraethoxy titanium, tetrapropoxy titanium, tetraisopropoxy titanium, tetrabutoxy titanium, tetraisobutoxy titanium, tetra-tert-butoxy titanium, tetracyclohexoxy titanium, tetraphenoxy titanium, tetratolyloxy titanium, tetrabenzyloxy titanium, and condensed products thereof. Among these titanium compounds, preferred are tetrapropoxy titanium, tetraisopropoxy titanium and tetrabutoxy titanium.

Further, as the titanium-based catalysts, in addition to the above titanium compounds represented by the general formula (2), there may also be used monoethanol amine titanate, diethanol amine titanate, triethanol amine titanate, etc.

In the present invention, these titanium-based catalysts may be used alone or in combination of any two or more thereof.

The tin-based catalysts usable in the present invention contain, for example,:
a tin compound represented by the general formula (3): 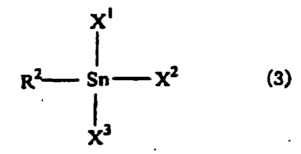 wherein R² is an alkyl group or an aryl group; and X¹ to X³ are each independently an alkyl group, an aryl group, an alkoxy group, an aryloxy group, an acyloxy group, a cycloalkyl group, a hydroxyl group or a halogen atom, or a condensed product thereof;
a tin compound represented by the general formula (4): 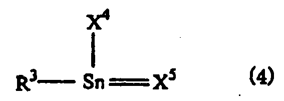 wherein R³ is an alkyl group or an aryl group; X⁴ is an alkyl group, an aryl group, an alkoxy group, an aryloxy group, an acyloxy group, a cycloalkyl group, a hydroxyl group or a halogen atom; and X⁵ is a sulfur atom or an oxygen atom, or a condensed product thereof
a tin compound represented by the general formula (5):

   X⁶-Sn-X⁷ (5)

   wherein X⁶ and X⁷ are each independently an acyloxy group, a hydroxyl group or a halogen atom, or a condensed product thereof; or SnO.

As the alkyl group as R² and X¹ to X³ in the general formula (3) and as R³ and X⁴ in the general formula (4), there my be used linear or branched alkyl groups having 1 to 20 carbon atoms. Specific examples of the alkyl groups include methyl, ethyl, propyl, isopropyl, various butyl groups, various pentyl groups, various hexyl groups, various octyl groups, various decyl groups, various dodecyl groups, various tetradecyl groups, various hexadecyl groups and various octadecyl groups. Examples of the aryl group include aryl groups having 6 to 20 carbon atoms which may have a substituent group bonded to an aromatic ring thereof. Specific examples of the aryl groups include phenyl, tolyl, xylyl and naphthyl.

Also, as the alkoxy group and the aryloxy group as X¹ to X³ in the general formula (3) and as X⁴ in the general formula (4), there may be used those alkoxy groups and aryloxy groups derived from the above-mentioned alkyl groups and aryl groups, respectively.

Further, as the acyloxy group as X¹ to X³ in the general formula (3), as X⁴ in the general formula (4) and as X⁶ and X⁷ in the general formula (5), there may be used aliphatic acyloxy groups having 2 to 20 carbon atoms, and aromatic acyloxy groups having 7 to 20 carbon atoms which may have a substituent group bonded to an aromatic ring thereof. Specific examples of the acyloxy groups include acetyloxy, propionyloxy, butyryloxy, hexanoyloxy, octanoyloxy, lauroyloxy, maleoyldioxy, fumaroyldioxy, benzoyloxy, phthaloyldioxy and salicyloyloxy. As the cycloalkyl group, there may be used cycloalkyl groups having 5 to 20 carbon atoms which may have a substituent group bonded to a ring thereof. Specific examples of the cycloalkyl groups include cyclopentyl, cyclohexyl, methyl cyclohexyl and cyclooctyl. The halogen atom includes fluorine, chlorine, bromine and iodine. Of these halogen atoms, preferred is chlorine.

In the present invention, as the tin-based catalyst, there may also be used condensed products of the tin compounds represented by the above general formulae (3), (4) and (5).

Specific examples of the tin-based catalyst usable in the present invention include dibutyl tin oxide, methylphenyl tin oxide, tetraethyl tin, hexaethyl tin oxide, cyclohexahexyl ditin oxide, didodecyl tin oxide, triethyl tin hydroxide, triphenyl tin hydroxide, triisobutyl tin acetate, dibutyl tin diacetate, diphenyl tin dilaurate, monobutyl tin trichloride, dibutyl tin dichloride, tributyl tin chloride, dibutyl tin sulfide, butylhydroxy tin oxide, tin octanoate, tin oxalate, tin chloride and tin oxide. In the present invention, these tin-based catalysts may be used alone or in combination of any two or more thereof. Further, one or more kinds of the tin-based catalysts may be used in combination with one or more kinds of the above titanium-based catalysts.

The amount of the transesterification reaction catalyst used is not particularly limited, and is usually from 0.01 to 10% by weight, preferably 0.05 to 5% by weight and more preferably 0.1 to 3% by weight based on the weight of the salicylic lower alkyl ester as the raw material.

The amounts of the salicylic lower alkyl ester and the alcohols or phenols used in the transesterification reaction are not particularly limited, and are preferably controlled to near stoichiometric amounts in view of a good yield and low costs. More specifically, the alcohols or phenols are advantageously used in an amount of usually about 0.7 to 1.7 mol, preferably 0.8 to 1.5 mol and more preferably 0.9 to 1.3 mol per mol of the salicylic lower alkyl ester.

The timing of addition of the transesterification reaction catalyst is not particularly limited. The transesterification reaction catalyst may be usually added immediately before initiation of the transesterification reaction, and/or may be added at any optional stage from initiation to completion of the transesterification reaction according to requirements.

The transesterification reaction may be conducted under a pressure ranging from an ordinary pressure to a reduced pressure up to about 13.3 kPa (100 mmHg). The reaction temperature may be usually selected from the range of from 80 to 220°C. In the present invention, in view of enhancement of reaction rate and suppression of undesirable side reactions, the transesterification reaction is preferably conducted at a temperature not lower than 130°C but less than 180°C, and more preferably at a temperature of from 140 to 170°C.

The transesterification reaction proceeds by removing lower alcohols liberated during the reaction out of the reaction system. Therefore, it is important that the reaction pressure and reaction temperature are respectively appropriately selected from such ranges capable of removing the lower alcohols out of the reaction system.

Although the reaction time varies depending upon kinds and amounts of the catalysts used as well as the reaction temperature and reaction pressure, the use of a reaction time of about 2 to 20 h is usually satisfactory. The transesterification reaction may also be performed in an atmosphere of an inert gas such as nitrogen, helium and argon, if desired.

In the present invention, the thus obtained reaction solution is subjected to distillation treatment in the presence of a basic compound or an epoxy compound having a boiling point higher than that of the salicylic ester as produced, either after being subjected to washing treatment in the case of using the basic catalyst such as sodium methoxide or potassium carbonate as the transesterification reaction catalyst, or directly without the washing treatment in the case of using the titanium-based catalyst or tin-based catalyst as the transesterification reaction catalyst.

The epoxy compound has the effect of capturing salicylic acid by produced during the reaction or distillation.

The timing of addition of epoxy compound is not particularly limited, and the basic compound or epoxy compound may be added either during or after the reaction as long as these compounds are present at least during the distillation treatment. In view of a good efficiency of addition of these compounds, the epoxy compound is preferably added before or during the distillation treatment and more preferably added to the reaction solution before the distillation treatment.

As the epoxy compound, there may be used various epoxy compounds which contain at least one epoxy group in a molecule thereof, and have a boiling point higher than that of the salicylic ester produced by the reaction.

Examples of the epoxy compound usable in the present invention include bisphenol-type epoxy resins such as bisphenol A (2,2-bis(4-hydroxyphenyl)propane]-type epoxy resins, bisphenol F (4,4'-dihydroxydiphenyl methane]-type epoxy resins, bisphenol S (4,4'-dihydroxydiphenyl sulfone]-type epoxy resins, bisphenol-type epoxy resins, tetramethyl bisphenol A-type epoxy resins, tetramethyl bisphenol F-type epoxy resins and tetramethyl bisphenol-type epoxy resins; novolak-type epoxy resins such as phenol novolak-type epoxy resins, cresol novolak-type epoxy resins and α-naphthol novolak-type epoxy resins; polyfunctional glycidyl ether-type epoxy resins such as trifunctional glycidyl ether-type epoxy resins and tetrafunctional glycidyl ether-type epoxy resins; aliphatic epoxy resins; alicyclic epoxy resins; imido-containing epoxy resins; and polyfunctional glycidyl amine-type epoxy resins.

In particular, the epoxy compounds have a boiling point higher than that of the salicylic ester as produced, since these compounds are prevented from being mixed in the aimed salicylic ester upon the distillation treatment. In addition, in view of effectively capturing the by-produced salicylic acid, there are more preferably used liquid epoxy compounds which contain two or more epoxy groups in a molecule thereof and are capable of being uniformly dissolved in the reaction solution to be distilled. The epoxy compound used in the present invention preferably has an epoxy equivalent of from 150 to 200.

These epoxy compounds may be used alone or in combination of any two or more thereof. The amount of the epoxy compound added is usually in the range of A x [(1.0 to 4.0)/100] g wherein A represents an epoxy equivalent of the epoxy compound used, per mol of the salicylic ester contained in the reaction solution to be distilled. When the epoxy compound is added in an amount of A x 1.5/100 g or larger, the by-produced salicylic acid can be sufficiently captured therewith.

Thus, the reaction solution to be distilled which contains the basic compound or epoxy compound is subjected to distillation treatment such as distillation under reduced pressure to recover unreacted alcohols or phenols as well as unreacted salicylic lower alkyl ester, and obtain a salicylic ester as a final product.

The distillation conditions are not particularly limited. The distillation may be conducted using an appropriate number of distillation columns. The conditions in the respective distillation apparatuses such as a temperature, a vacuum degree and a reflux ratio may be appropriately determined according to kinds of unreacted alcohols or phenols and unreacted salicylic lower alkyl ester to be distilled therefrom as well as the salicylic ester as the aimed product.

Thus, in the present invention, the reaction solution is subjected to distillation treatment in the presence of the basic compound or epoxy compound as a capturing agent for capturing the by-produced salicylic acid. As a result, in processes subsequent to the distillation process, precipitation of the salicylic acid in apparatuses used therein can be effectively prevented.

In accordance with the present invention, there is provided an industrially useful process for producing salicylic esters by transesterification reaction in which precipitation of salicylic acid in apparatuses is effectively suppressed in processes subsequent to distillation process thereof, so that problems such as deteriorated cooling efficiency of a condenser and clogging in conduits due to the precipitation of salicylic acid as well as a large load for cleaning facilities after completion of the production owing to these problems can be prevented.

The salicylic esters obtained according to the process of the present invention are useful as raw materials for perfumes.

The following examples further describe and demonstrate embodiments of the present invention, The examples are given only for the purpose of illustration and are not to be construed as limitations of the present invention.

### EXAMPLE 1

Into a four-necked glass flask were charged 198.87 g (1.30 mol) of methyl salicylate and 169.27 g (1.69 mol) of cyclohexanol, and further 2.29 g (0.50 mol%) of di-n-butyl tin diacetate as a catalyst was dropped thereinto under. stirring. After completion of the addition, the temperature of the resultant mixture was raised while distilling off methanol liberated out of the reaction system.

After the temperature of the reaction solution reached 170°C, the reaction solution was held at that temperature under heating. Successively, while distilling off methanol liberated out of the reaction system, the contents of the flask were reacted with each other for 10 h.

After the resultant reaction solution was cooled, 250.3 g of the reaction solution was mixed with 1.85 g of an epoxy compound "AER250" (registered trademark) available from Asahi Kasei Co., Ltd.; epoxy equivalent: 184 g/eq), and the obtained mixture was subjected to distillation treatment using a 10-stage Sulzer-type distillation column. First, 35.7 g of unreacted cyclohexanol was distilled off under a top pressure of 1.33 kPa at a top temperature of 59 to 64°C and a reflux ratio of 3, and then 16.2 g of methyl salicylate was distilled off at a top temperature of 64 to 160°C and a reflux ratio of 3. Further, cyclohexyl salicylate was distilled off at a top temperature of 160 to 161°C, thereby recovering 178.4 g of the cyclohexyl salicylate.

As a result, it was confirmed that both during and after the distillation treatment, neither precipitation of salicylic acid in the distillation apparatus, nor clogging in the apparatus occurred. In addition, a fraction obtained by the distillation treatment as well as residues obtained by washing an inside of the apparatus with acetone after the distillation treatment were subjected to gas chromatographic analysis. As a result, it was confirmed that no salicylic acid was detected in the fraction and residues (detection limit: 0.01%).

### EXAMPLE 2

The transesterification reaction was conducted in the same manner as in Example 1 except for using tetraisopropoxy titanium as the catalyst in place of di-n-butyl tin diacetate.

More specifically, into a four-necked glass flask were charged 165.50 g (1.65 mol) of methyl salicylate and 228.52 g (1.50 mol) of cyclohexanol, and further 2.16 g (0.51 mol%) of tetraisopropoxy titanium as the catalyst was dropped thereinto under stirring. After completion of the addition, the temperature of the resultant mixture was raised while distilling off methanol liberated out of the reaction system.

After the temperature of the reaction solution reached 170°C, the reaction solution was held at that temperature under heating. Successively, while distilling off methanol liberated out of the reaction system, the contents of the flask were reacted with each other for 10 h.

After the resultant reaction solution was cooled, 330.3 g of the reaction solution was mixed with 2.32 g of an epoxy compound "AER250" (registered trademark) available from Asahi Kasei Co., Ltd.; epoxy equivalent: 184 g/eq), and the obtained mixture was subjected to distillation treatment using a 10-stage Sulzer-type distillation column. First, 34.7 g of unreacted cyclohexanol was distilled off under a top pressure of 1.33 kPa at a top temperature of 61°C and a reflux ratio of 1, and then 24.9 g of methyl salicylate was distilled off under a top pressure of 1.33 kPa at a top temperature of 61 to 162°C and a reflux ratio of 10. Further, cyclohexyl salicylate was distilled off under a top pressure of 1.33 kPa at a top temperature of 162 to 163°C, thereby recovering 237.6 g of the cyclohexyl salicylate.

As a result, it was confirmed that both during and after the distillation treatment, neither precipitation of salicylic acid in the distillation apparatus, nor clogging in the apparatus occurred. In addition, similarly to Example 1, a fraction obtained by the distillation treatment as well as residues obtained by washing an inside of the apparatus with acetone after the distillation treatment were subjected to gas chromatographic analysis. As a result, it was confirmed that no salicylic acid was detected in the fraction and residues (detection limit: 0.01%).

### COMPARATIVE EXAMPLE 1

The reaction solution was subjected to distillation treatment in the same manner as in Example 2 except for adding no epoxy compound (AER250) thereto. As a result, it was confirmed that white solids (salicylic acid) were precipitated at a top of the distillation column during the distillation treatment after the stage where methyl salicylate was distilled off, and clogging was caused thereat. At this time, a fraction obtained by the distillation treatment was subjected to gas chromatographic analysis. As a result, it was confirmed that 2.5% salicylic acid was detected in the fraction.

## Claims

1. A process for producing a salicylic ester, comprising: subjecting a transesterification reaction solution obtained by transesterifying a salicylic C₁₋₄ alkyl ester with alcohols or phenols in the presence of at least one catalyst selected from the group consisting of titanium-based catalysts, tin-based catalyst, and aluminium-based catalysts, to distillation treatment in the presence of an epoxy compound having a boiling point higher than that of the salicylic ester as produced.

2. The process according to claim 1, wherein said titanium-based catalyst contains at least one selected from the group consisting of a titanium compound represented by the following general formula (2):
Ti(OR¹)₄ (2)
wherein R¹ is an alkyl group, a cycloalkyl group, an aryl group or an arylalkyl group,
and a condensed product thereof.

3. The process according to claims 1 or 2, wherein the tin-based catalyst contains at least one selected from the group consisting of a tin compound represented by the general formula (3): 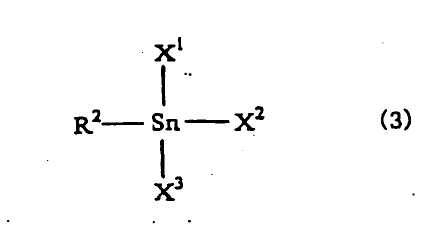 wherein R² is an alkyl group or an aryl group; and X¹ to X³ are each independently an alkyl group, an aryl group, an alkoxy group, an aryloxy group, an acyloxy group, a cycloalkyl group, a hydroxyl group or a halogen atom, and a condensed product thereof;
a tin compound represented by the general formula (4): 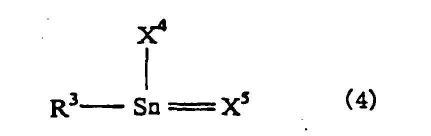 wherein R³ is an alkyl group or an aryl group X⁴ is an aryl group, an alkoxy group, an aryloxy group, an acyloxy group, a cycloalkyl group, a hydroxyl group or a halogen atom; and X⁵ is a sulfur atom or an oxygen atom,
and a condensed product thereof;
a tin compound represented by the general formula (5):
X⁶-Sn-X⁷ (5) (5)
wherein X⁶ and X⁷ are each independently an acyloxy group, a hydroxyl group or a halogen atom, and a condensed product thereof;
and SnO.

4. The process according to anyone of claims 1 to 3, wherein said epoxy compound contains two or more epoxy groups in a molecule thereof.

5. The process according to any one of claims 1 to 4, wherein the salicylic ester is represented by the general formula (1): 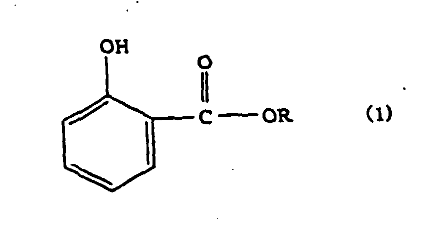 wherein R is a saturated or unsaturated, chain-like aliphatic or cyclic aliphatic group having 3 to 20 carbon atoms, a heterocyclic group having 3 to 20 carbon atoms, an aryl group having 6 to 20 carbon atoms, or an arylalkyl group having 7 to 20 carbon atoms.

6. The process according to any one of claims 1 to 5, wherein the salicylic ester is cyclohexyl salicylate, hexyl salicylate, pentyl salicylate, isopentyl salicylate, cis-3-hexenyl salicylate or benzyl salicylate.

## Patentansprüche

1. Verfahren zur Erzeugung eines Salicylsäureesters, umfassend die Durchführung einer Destillationsbehandlung mit einer Umesterungsreationslösung, erhalten durch Umestern eines Salicyl-C₁₋₄-alkylesters mit Alkoholen oder Phenolen in der Gegenwart von zumindest einem Katalysator, ausgewählt aus der Gruppe bestehend aus Katalysatoren auf Titanbasis, Zinnbasis und Aluminiumbasis, in der Gegenwart einer Epoxyverbindung mit einem höheren Siedepunkt als der erzeugte Salicylester.

2. Verfahren nach Anspruch 1, worin der Katalysator auf Titanbasis zumindest einen enthält, ausgewählt aus der Gruppe bestehend aus einer Titanverbindung mit der folgenden allgemeinen Formel (2):
Ti(OR¹)₄ (2)
worin R¹ eine Alkylgruppe, Cycloalkylgruppe, Arylgruppe oder Arylalkylgruppe ist, und einem kondensierten Produkt davon.

3. Verfahren nach Anspruch 1 oder 2, worin der Katalysator auf Zinnbasis zumindest einen enthält, ausgewählt aus der Gruppe bestehend aus einer Zinnverbindung mit der allgemeinen Formel (3): 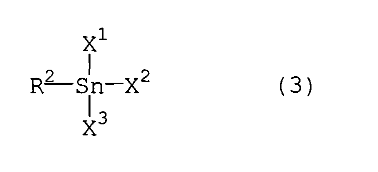 worin R² eine Alkylgruppe oder Arylgruppe ist und X¹ bis X³ jeweils unabhängig eine Alkylgruppe, Arylgruppe, Alkoxygruppe, Aryloxygruppe, Acyloxygruppe, Cycloalkylgruppe, Hydroxylgruppe oder Halogenatom sind, und einem kondensierten Produkt davon;
Zinnverbindung mit der allgemeinen Formel (4): 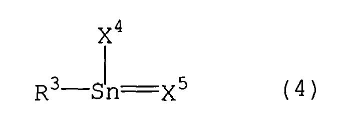 worin R³ eine Alkylgruppe oder Arlygruppe ist, X⁴ eine Arylgruppe, Alkoxygruppe, Aryloxygruppe, Acyloxygruppe, Cycloalkylgruppe, Hydroxylgruppe oder Halogenatom ist, und X⁵ ein Schwefelatom oder Sauerstoffatom ist, und einem kondensierten Produkt davon,
Zinnerverbindung mit der allgemeinen Formel (5):
X⁶-Sn-X⁷ (5)
worin X⁶ und X⁷ jeweils unabhängig eine Acyloxygruppe, Hydroxylgruppe oder Halogenatom sind, und ein kondensiertes Produkt davon;
und SnO.

4. Verfahren nach einem der Ansprüche 1 bis 3, worin die Epoxyverbindung zwei oder mehrere Epoxygruppen in einem Molekül davon enthält.

5. Verfahren nach einem der Ansprüche 1 bis 4, worin der Salicylester durch die allgemeine Formel (1) dargestellt ist: 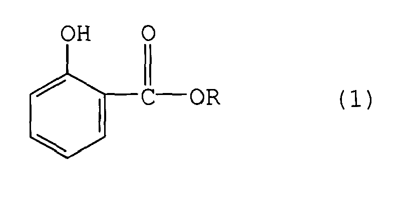 worin R eine gesättigte oder ungesättigte, kettenartige aliphatische oder cyclische aliphatische Gruppe mit 3 bis 20 Kohlenstoffatomen, heterocyclische Gruppe mit 3 bis 20 Kohlenstoffatomen, Arylgruppe mit 6 bis 20 Kohlenstoffatom oder Arylalkylgruppe mit 7 bis 20 Kohlenstoffatomen ist.

6. Verfahren nach einem der Ansprüche 1 bis 5, worin der Salicylester Cyclohexylsalicylat, Hexylsalicylat, Pentylsalicylat, Isopentylsalicylat, cis-3-Hexenylsalicylat oder Benzylsalicylat ist.

## Revendications

1. Procédé pour produire un ester salicylique, comprenant : de soumettre une solution de réaction de trans-estérification obtenue par trans-estérification d'un ester d'alkyl(en C₁₋₄)salicylique avec des alcools ou des phénols en présence d'au moins un catalyseur choisi dans le groupe constitué par des catalyseurs à base de titane, des catalyseurs à base d'étain, et des catalyseurs à base d'aluminium, à un traitement de distillation en présence d'un composé époxy ayant un point d'ébullition supérieur à celui de l'ester salicylique produit.

2. Procédé selon la revendication 1, dans lequel ledit catalyseur à base de titane contient au moins l'un choisi dans le groupe constitué par un composé de titane représenté par la formule générale suivante (2) :
Ti(OR¹)₄ (2)
dans laquelle R¹ est un groupe alkyle, un groupe cycloalkyle, un groupe aryle ou un groupe arylalkyle, et un produit condensé celui-ci.

3. Procédé selon les revendications 1 ou 2, dans lequel le catalyseur à base d'étain contient au moins l'un choisi dans le groupe constitué d'un composé d'étain représenté par la formule générale (3) : 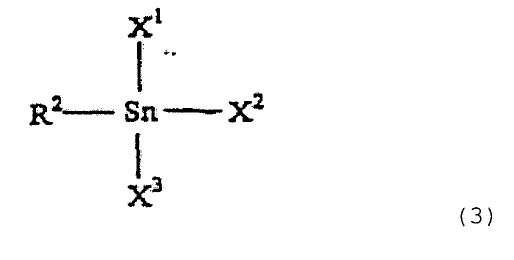 dans laquelle R² est un groupe alkyle ou un groupe aryle, et X¹ à X³ sont chacun indépendamment un groupe alkyle, un groupe aryle, un groupe alcoxy, un groupe aryloxy, un groupe acyloxy, un groupe cycloalkyle, un groupe hydroxyle ou un atome d'halogène, et un produit condensé celui-ci, un composé d'étain représenté par la formule générale (4) : 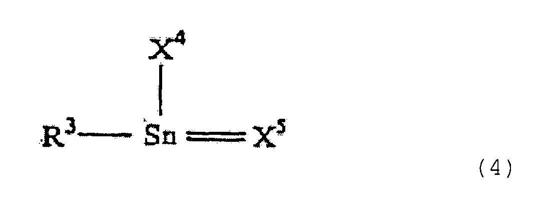 dans laquelle R³ est un groupe alkyle ou un groupe aryle, X⁴ est un groupe aryle, un groupe alcoxy, un groupe aryloxy, un groupe acyloxy, un groupe cycloalkyle, un groupe hydroxyle ou un atome d'halogène, et X⁵ est un atome de soufre ou un atome d'hydrogène,
et un produit condensé celui-ci,
un composé d'étain représenté par la formule générale (5) :
X⁶-Sn-X⁷ (5)
dans laquelle X⁶ et X⁷ sont chacun indépendamment un groupe acyloxy, un groupe hydroxyle ou un atome d'halogène, et un produit condensé celui-ci,
et SnO.

4. Procédé selon l'une quelconque des revendications 1 à 3, dans lequel ledit composé époxy contient deux ou plus groupes époxy dans une molécule de celui-ci.

5. Procédé selon l'une quelconque des revendications 1 à 4, dans lequel l'ester salicylique est représenté par la formule générale (1) : 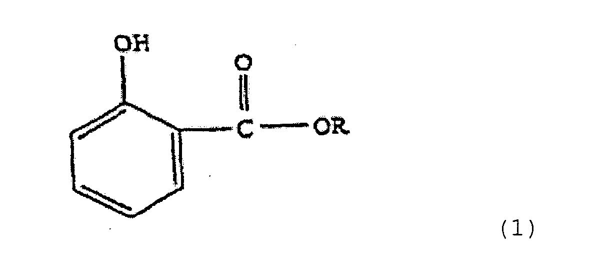 dans laquelle R est un groupe aliphatique cyclique ou aliphatique du type chaîne, saturé ou insaturé ayant 3 à 20 atomes de carbone, un groupe hétérocyclique ayant 3 à 20 atomes de carbone, un groupe aryle ayant 6 à 20 atomes de carbone, ou un groupe arylalkyle ayant 7 à 20 atomes de carbone.

6. Procédé selon l'une quelconque des revendications 1 à 5, dans lequel l'ester salicylique est le salicylate de cyclohexyle, le salicylate d'hexyle, le salicylate de pentyle, le salicylate d'isopentyle, le salicylate de cis-3-hexényle ou le salicylate de benzyle.
